# EUROPEAN PATENT APPLICATION

(11) **EP 4 837 549 A1**
(43) Date of publication of application: **11.11.2026**
(21) Application number: 25174918.0
(22) Date of filing: 07.05.2025
(51) Int. Cl.: A61F 13/15, A61F 13/535, A61F 13/537

(54) **ABSORBENT ARTICLE COMPRISING AN ACQUISITION LAYER AND A CUSHION LAYER MADE OF THE SAME MATERIAL**

(71) Applicant: The Procter & Gamble Company, Cincinnati, OH 45202 (US)
(72) Inventor: ROSAR, Markus, 65824 Schwalbach am Taunus (DE); MISHRA, Piyush, Akashi, Hyogo, 674-0093 (JP); LEE, DonSub, Akashi, Hyogo, 674-0093 (JP)
(74) Representative: P&G Patent Germany

(57) **Abstract**

A personal hygiene absorbent article (20) comprising a liquid permeable topsheet (24) an absorbent core (28), a backsheet (25), an acquisition layer (52) disposed between the topsheet and the absorbent core, a cushion layer (60) disposed between the absorbent core and the backsheet, or a cushion layer (60') disposed between the absorbent layer and the core wrap bottom layer, wherein the acquisition layer and the cushion layer are made of the same bi-functional material. Processes for making these articles comprising continuously slitting a bi-functional material along its length to form an acquisition material and a cushion material.

## Description

### FIELD OF THE INVENTION

The invention relates to absorbent articles for personal hygiene, such as baby diapers and adult incontinence products, and a process for making the same.

### BACKGROUND OF THE INVENTION

Disposable absorbent articles for babies, toddlers and incontinent adults have evolved over the years to be evermore reliable and affordable. These articles typically comprise a liquid permeable topsheet on the wearer-facing side, a liquid impermeable backsheet on the garment-facing side, and an absorbent system between these two layers among other components. The absorbent system typically comprises an acquisition layer underneath the topsheet and an absorbent core between the acquisition layer and the backsheet.

The absorbent cores used in diapers require relatively large capacity and thus nowadays typically comprise an absorbent layer comprising or consisting of superabsorbent polymers (SAP) sandwiched between a core wrap top layer and a core wrap bottom layer. Urine passes through the topsheet, is acquired by the acquisition layer and is absorbed by the absorbent layer in the absorbent core. The superabsorbent polymer particles in the absorbent layer may be mixed with cellulose fibers (in the context of absorbent articles often referred to as "fluff pulp" or "airfelt"). In general, there is a trend for absorbent layers comprising ever higher proportion of superabsorbent polymer particles relative to cellulose fibers. Absorbent layers free of cellulose fibers ("airfelt-free" cores) have also been used in commercial products. However this may have a negative impact in the speed of acquisition of the fluid by the absorbent core, as the SAP requires some time to absorb the fluid.

Various absorbent article designs have been suggested to remediate this issue. Absorbent material-free channels have been incorporated in the absorbent layer to improve fluid distribution, see e.g. WO2012/170778A1, WO2012/170781 (Kreuzer et al.). It has also been suggested to incorporate a lower acquisition and distribution layer between the layer of absorbent material and the backsheet, see WO2021/118904A1 (Grenier et al.) to reduce the amount of liquid in a zone corresponding to 800 µm starting from and including the backsheet.

Adding an additional layer to a diaper construction increases manufacturing costs directly because of capital spending required for the extra equipment required such as unwinder, and indirectly due to labor cost needed for refilling the raw material on the converting line. Modern converting lines are extremely fast and require regular replenishment, which is relatively labor intensive in otherwise highly automatized converting lines, in particular when the material, such as nonwoven materials, is provided in relatively heavy and large rolls.

There is thus a need for absorbent articles having improved liquid handling and tactile properties and process for making these articles more economically.

### SUMMARY OF THE INVENTION

The invention is directed to a personal hygiene absorbent article extending in a longitudinal direction and a transversal direction, the absorbent article comprising:
- a liquid permeable topsheet;
- a backsheet;
- an absorbent core between the backsheet and the topsheet, the absorbent core comprising a core wrap having a top layer and a bottom layer and an absorbent layer, wherein the absorbent layer is between the core wrap top layer and the core wrap bottom layer, wherein the absorbent layer comprises or consists of superabsorbent particles;
- an acquisition layer disposed between the topsheet and the absorbent core;
- a cushion layer, wherein the cushion layer is either disposed between the absorbent core and the backsheet, or between the absorbent layer and the core wrap bottom layer.

According to the invention, the acquisition layer and the cushion layer are made of the same bi-functional material that provides at the same time cushioning and liquid handling to the article.

The invention is also directed to a process for making the absorbent articles in a converting line having a machine direction (MD) and a cross-direction (CD), said process comprising the steps of:
- providing a web of bi-functional material;
- slitting the web of bi-functional material along its length to form an acquisition material and a cushion material;
- cutting the acquisition material and cushion material in cross-direction into discrete acquisition layers and cushion layers;
- assembling an acquisition layer and a cushion layer with a topsheet, backsheet, absorbent core and any other components of the absorbent article to make discrete absorbent articles as indicated previously.

Advantageous features and properties of the invention are further detailed in the attached claims and the following description.

### BRIEF DESCRIPTION OF THE DRAWINGS

While the specification concludes with claims particularly pointing out and distinctly claiming the present invention, it is believed that the same will be better understood from the following description read in conjunction with the accompanying drawings in which:
Fig. 1 is an exemplary absorbent article in the form of a taped diaper, with some inner layers partially revealed.
Fig. 2 is a transversal cross-section of the diaper of Fig. 1, wherein the cushion layer is disposed between the core wrap bottom layer and the backsheet.
Fig. 3 is an alternative transversal cross-section of the diaper of Fig. 1, wherein the cushion layer is disposed within the absorbent core between the absorbent layer and the core wrap bottom layer.
Fig. 4 is a schematic perspective view of a slitting device used to slit the bi-functional material web along its length to form an acquisition material and a cushion material.
Fig. 5 is a schematic showing a converting process according to the invention for making articles as in Figs. 1-2, where the cushion layer is disposed between the absorbent core and the backsheet.
Fig. 6 is a schematic showing an alternative converting process according to the invention for making articles as in Fig. 5 where the cushion layer is disposed within the absorbent core between the absorbent layer and the core wrap bottom layer.
Fig. 7 is a schematic showing how the absorbent system produced in Fig. 5 or Fig. 6 can be further assembled with a topsheet composite material and a backsheet material to form the absorbent article.

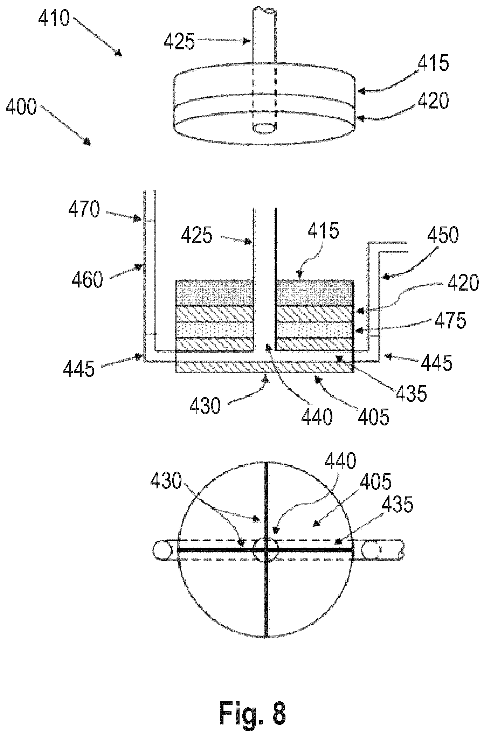

Figs. 8-9 show partially schematic views of an equipment assembly used in the In-Plane Radial Permeability (IPRP) Test described herein.

### DETAILED DESCRIPTION OF THE INVENTION

### Definitions

As used herein, "absorbent article" refers to devices that absorb and contain body exudates, and, more specifically, refers to devices that are placed against or in proximity to the body of the wearer to absorb and contain any of the various exudates discharged from the body. Absorbent articles include diapers (baby and infant diapers as well as diapers for adult incontinence), pants (for babies, infants and for adults), absorbent inserts (which are intended to be inserted into an outer cover to form a diaper or pant), feminine care absorbent articles such as sanitary napkins and pantiliners, and the like. As used herein, the term "exudates" includes, but is not limited to, urine, blood, vaginal discharges, sweat and fecal matter. The absorbent articles of the present invention may be disposable absorbent articles, in particular disposable taped diapers and disposable pant diapers.

As used herein, "diaper" refers to an absorbent article generally worn by babies, infants and incontinent adults about the lower torso so as to encircle the waist and legs of the wearer and that is specifically adapted to receive and contain urinary and fecal waste. In a diaper pant, the longitudinal edges of the first and second waist region are attached to each other so that waist opening and leg openings are pre-formed. A diaper pant is placed in position on the wearer by inserting the wearer's legs into the leg openings and sliding the pant into position about the wearer's lower torso similarly to an underwear.

A pant may be pre-formed by any suitable technique including, but not limited to, joining together portions of the absorbent article using re-fastenable and/or non-refastenable bonds (e.g., seam, weld, adhesive, cohesive bond, fastener, etc.). A pant may be pre-formed anywhere along the circumference of the article (e.g., side fastened, front waist fastened).

In a taped diaper, the waist opening, and leg openings are formed when the diaper is applied onto a wearer by (releasably) attaching the longitudinal edges of the first and second waist region to each other on both sides by a suitable fastening system.

As used herein, the terms "nonwoven", "nonwoven web" and "nonwoven layer" are used interchangeably. Nonwovens are engineered fibrous assemblies, primarily planar, which have been given a designed level of structural integrity by physical and/or chemical means, excluding weaving, knitting or paper making. The fibers may be of natural origin, such as cotton or bamboo fibers, or man-made origin. Synthetic fibers are typically made of polyolefins (such as polyethylene, polypropylene or combinations and mixtures thereof), polyethylene terephthalate (PET), co-PET, polylactic acid (PLA), polyhydroxy alkanoid (PHA), or mixtures or combinations thereof. The fibers may be staple fibers (e.g. in carded nonwovens) or continuous fibers (e.g. in spunbonded or meltblown nonwovens).

Nonwovens may be formed by many known processes such as meltblowing, spunlaying, solvent spinning, electrospinning and carding. The fibrous web can be consolidated, e.g. by hydroentanglement (in spunlaced nonwovens), air-through bonding (using hot air that is blown through the fiber layer in the thickness direction), needle-punching, one or more patterns of bonds and bond impressions created through localized compression and/or application of heat or ultrasonic energy, or a combination thereof. The fibers may also be consolidated by a binder. The binder may be binder fibers (which are subsequently molten) or may be a liquid such as a styrene butadiene latex binder. A liquid binder is provided to the fibers (e.g. by spraying, printing or foam application) and is subsequently cured to solidify. The basis weight of nonwovens is usually expressed in grams per square meter (g/m²).

"Hydrophilic" describes surfaces of substrates which are wettable by aqueous fluids (e.g., aqueous body fluids) deposited on these substrates. Hydrophilicity and wettability are typically defined in terms of contact angle and the strike-through time of the fluids, for example through a nonwoven fabric. This is discussed in detail in the American Chemical Society publication entitled "Contact Angle, Wettability and Adhesion", edited by Robert F. Gould (Copyright 1964). A surface of a substrate is said to be wetted by a fluid (i.e., hydrophilic) when either the contact angle between the fluid and the surface is less than 90°, or when the fluid tends to spread spontaneously across the surface of the substrate, both conditions are normally co-existing. Conversely, a substrate is considered to be "hydrophobic" if the contact angle is greater than 90° and the fluid does not spread spontaneously across the surface of the fiber.

"Longitudinal" refers to a direction (y) running substantially perpendicular from a waist edge to an opposing waist edge of the article and generally parallel to the maximum linear dimension of the article. "Transversal" refers to a direction (x) perpendicular to the longitudinal direction.

"Inner" and "outer" refer respectively to the relative location of an element or a surface of an element or group of elements. "Inner" implies the element or surface is oriented towards the inside of the article in particular towards the absorbent layer, while "Outer" implies the element or surface is oriented towards the outside of the article, in particular away from the absorbent layer.

"Body-facing" and "garment-facing" refers respectively to the relative location of an element or a surface of an element or group of elements. "Body-facing" implies the element or surface is nearer to the wearer during wear than another element of the same component. "Garment-facing" implies the element or surface is more remote from the wearer during wear than another element of the same component. The garment-facing surface may face another (i.e. other than the wearable article) garment of the wearer, other items, such as the bedding, or the atmosphere.

"Comprise," "comprising," and "comprises" are open ended terms, each specifies the presence of the feature that follows, e.g. a component, but does not preclude the presence of other features, e.g. elements, steps, components known in the art or disclosed herein. These terms based on the verb "comprise" encompasses the narrower terms "consisting essential of" which excludes any element, step or ingredient not mentioned which materially affect the way the feature performs its function, and the term "consisting of" which excludes any element, step, or ingredient not specified.

"Machine direction" or "MD" in the context of an absorbent article making process refers to the general direction in which the materials, such as non-woven fabrics, absorbent cores, and other components of the diaper, are fed into the machinery and processed. "Cross-machine direction" or "CD" refers to the orientation that is perpendicular to the machine direction (MD).

### General description of an exemplary diaper

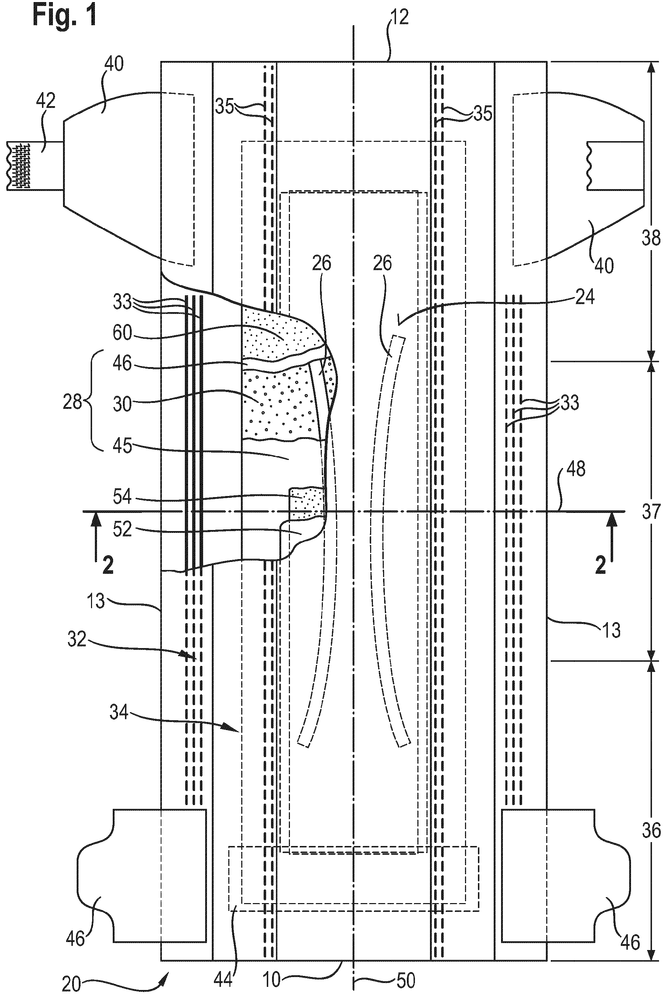

Figure 1 is a plan view of an exemplary taped diaper 20, in a flat-out state, with portions of the diaper being cut-away to show the construction of the diaper more clearly. This diaper 20 is shown for illustration purpose only as the structure of the present invention may be comprised in a wide variety of diapers or other absorbent articles, such as pants having pre-formed side seams. The side seams of pant articles can be opened by cutting or otherwise, if it is desired to place the pant in a flattened-out configuration.

As illustrated in Figure 1-2, the absorbent article comprises a topsheet 24, backsheet 25, and an absorbent layer 30 which is positioned between the topsheet 24 and the backsheet 25. The absorbent layer 30 has a pre-determined shape as considered in the plane formed by the transversal direction x and longitudinal direction y, when the article is flattened-out. The absorbent layer may be substantially rectangular as illustrated in Fig. 1. The absorbent layer may also be non-rectangular shaped, such as having tapered longitudinal edges as in a sand-hour or dog bone shape. The absorbent layer 30 may further comprise longitudinally-oriented channels 26. The core channels are typically areas substantially free of absorbent material within the absorbent layer, and that facilitate the distribution of a fluid along the length of the absorbent article.

Absorbent articles typically comprise an acquisition layer 52 directly underneath the topsheet. An example of acquisition layer material is a surfactant-treated, latex-bonded nonwoven. The absorbent article can optionally a distribution layer 54 between the acquisition layer and the absorbent core, especially when the absorbent material of the absorbent core is free of cellulose fibers. A distribution layer that may be used consist of cross-linked cellulose fibers, as is known in the art. The prior art discloses many types of acquisition-distribution systems, see for example WO2000/59430, WO95/10996, US5700254, WO02/067809.

The absorbent article 20 may also comprise inner barrier leg cuffs 34 and outer leg cuffs 32, as is known in the art. The inner barrier cuffs 34 can extend upwards from the surface of the article to provide retention of the waste, while the outer cuffs are typically formed in the plane of the chassis of the article as defined by topsheet and backsheet. These cuffs are preferably elasticized, as is known in the art. The cuffs thus preferably comprise elastic threads 33, 35. Moreover, the absorbent article may comprise a fastening system, such as an adhesive fastening system or a hook and loop fastening member. The fastening system can comprise tape tabs 42 disposed on back ears 40, such as adhesive tape tabs or tape tabs comprising hook elements, cooperating with a landing zone 44 (e.g. a nonwoven web providing loops in a hook and loop fastening system). While taped diapers typically comprise back ears 40, and front ears 46, these are typically not present in pant-type absorbent articles having pre-formed side seams.

The front and/or back ears 40, 46 may be separate components attached to the absorbent article or may instead be continuous with portions of the topsheet and/or backsheet such that these portions form all or a part of the front and/or back ears 40, 46. Also combinations of the aforementioned are possible, such that the front and/or back ears 40, 46 are formed by portions of the topsheet and/or backsheet while additional materials are attached to form the overall front and/or back ears 40, 46. The front and/or back ears may be elastic or non-elastic. Also, the front ears 46 may be applied as separate components attached to the absorbent article while the back ears (or parts thereof) 40 may be continuous with portions of the backsheet and/or topsheet - or vice versa.

The longitudinal centerline 80 is the imaginary line separating the diaper along its length in two equal halves. The transversal centerline 90 is the imagery line perpendicular to the longitudinal line 80 in the plane of the flattened-out diaper and going through the middle of the length of the diaper (the same applies to for the transversal centerline and longitudinal line of other absorbent articles of the present invention). The periphery of the diaper 20 is defined by the outer edges of the diaper. The longitudinal edges 13 of the diaper may run generally parallel to the longitudinal centerline 80 of the diaper 20 and the end edges (the front waist edge 10 and the back waist edge 12) run between the longitudinal edges generally parallel to the transversal centerline 90 of the diaper 20.

The absorbent article, whether diaper or pant, can be notionally divided in front waist region 36, a back waist region 38 opposed to the first waist region 36 and a crotch region 37 located between the front waist region 36 and the back waist region 38. The front region, the crotch region and the back region each constitutes a third of the absorbent article as measured along the longitudinal centerline.

Further, the absorbent article may comprise other optional but conventional elements, which are not represented for simplicity, such as an elastic back waist feature (cuff or band), a front waist elastic feature (band), a lotion applied onto the body-facing surface of the topsheet, or a urine indicator disposed on the inner side of the backsheet that changes color when contacted with urine.

The topsheet 24, the backsheet 25 and the absorbent layer 30 may be assembled in a variety of well-known configurations, in particular by gluing, heat embossing, ultrasonic bonding or combinations thereof. Exemplary diaper configurations are described generally in US3,860,003; US5,221,274; US5,554,145; US5,569,234; US5,580,411 and US6,004,306.

The main components of the articles according to the invention are now discussed in more details below.

### Topsheet

The topsheet 24 is a liquid permeable layer that is in contact with the wearer's skin permitting liquid bodily exudates to readily penetrate into the absorbent article. Typical topsheet material are nonwovens and apertured plastic films, but other materials are possible such as porous foams, reticulated foams, woven materials etc... Suitable nonwovens may comprise natural fibers (e.g., wood or cotton fibers), synthetic fibers (e.g., polypropylene or bicomponent PE/PP fibers or mixtures thereof), or a combination of natural and synthetic fibers. The topsheet may consist of a single layer, or may be a laminate or a composite of different materials. The topsheet may be apertured or non-apertured. The topsheet may also have three-dimensional features, and/or may have a plurality of embossments (e.g., a bond pattern). Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

### Backsheet

The backsheet 25 is generally that portion of the absorbent article 20 that constitutes all or a part of the garment-facing surface of the absorbent article. The backsheet 25 may be joined at least partially to the topsheet 24, the absorbent layer 30, the substrate layer 46, or the cushion layer 60, by any attachment methods known to those of skill in the art in particular adhesive and/or fusion and/or ultrasonic bonding. The backsheet 25 prevents, or at least inhibits, the bodily exudates absorbed and contained in the absorbent layer 30 from soiling articles such as bedsheets, undergarments, and/or clothing. The backsheet is typically liquid impermeable, or at least substantially liquid impermeable.

The backsheet 25 is typically comprised of a thin impermeable plastic film, usually a thermoplastic film having a thickness of about 0.01 mm to about 0.05 mm. The backsheet film material may be breathable, which permit vapors to escape from the absorbent article, while still preventing, or at least inhibiting, bodily exudates from passing through the backsheet. A breathable backsheet may have a Water Vapor Transmission Rate (WVTR) of from 1,000 to 15,000 g/m²/24h, or from 1,000 to 10,000 g/m²/24h, or from 1,500 to 10,000 g/m²/24h as measured using a PERMATRAN-W Model 101K (available from Mocon, Inc., Minneapolis, MN) or equivalent, according to Nonwovens Standard Procedure NWSP 70.4.R0(15) with the following specifications: experiments were carried out in a lab controlled at 23 °C ± 2 C° and 50 %RH ± 2 %RH and the instrument cells heated to 37.8°C (100°F).

The backsheet 25 may also comprise a backsheet outer cover nonwoven (not represented separately) to improve the touch of the backsheet. The backsheet outer cover nonwoven is typically a thin nonwoven material that is adhesively joined to the outer surface of the backsheet film. The backsheet outer cover nonwoven forms the outermost garment-facing surface of the backsheet. The backsheet outer cover nonwoven may comprise a bond pattern, apertures, and/or three-dimensional features.

### Absorbent core 28

The absorbent layer 30 typically comprises superabsorbent polymer in the form of particles. The superabsorbent polymer particles may be mixed with cellulose fibers. Alternatively, the absorbent layer may be free of cellulose fibers mixed with the superabsorbent polymer particles. Such absorbent cores where the superabsorbent polymer particles are not mixed with cellulose fibers are designated in the art as airfelt-free cores. In such airfelt-free cores, the superabsorbent polymers particles may be immobilized by a glue on a core substrate, in particular a microfibrous glue, or alternatively supported within a high loft, porous nonwoven, as is known in the art.

The absorbent layer 30 is typically disposed between a core wrap top layer 45 oriented towards the topsheet and a core wrap bottom layer 46 oriented towards the backsheet. The absorbent core is defined by the core wrap and the absorbent material within the core wrap, and optionally comprising a cushion layer 60' between the absorbent layer the bottom core wrap layer. The core wrap top layer and core wrap bottom layer are commonly referred to as, respectively, core cover and dusting layer, and together as core wrap. The core wrap layers are typically low basis weight nonwoven, (typically in the range of from 10 gsm to 20 gsm, in particular less than 14 gsm). Core wrap layers are typically SMS nonwovens (Spunbond-Meltblown-Spunbond laminate) are known in the art. The core wrap top and bottom layers 45, 46 may be any material capable of providing a support for the absorbent layer.

The core wrap layers may be made of the same or different materials, i.e. two nonwoven webs which have the same of different properties. The upper and the lower substrate layers 45, 46 may be made of two discrete layers, as represented in the Figures. The substrate layers are preferably bonded longitudinally to prevent the absorbent layer from being released sideways. The substrate layers may also be optionally bonded transversally at the front and the back of the absorbent core. The substrate layers may be bonded in a C-wrap configuration where one of the top or bottom substrate layer is larger than the other, so that flaps can be folded around the absorbent layer and attached to the other substrate, but other bonding configurations are possible, in particular a face to face bonding configuration. Alternatively, the core wrap may be made of a single material, i.e. of the same nonwoven, which is wrapped around the absorbent layer.

The core wrap top layer 45 and the core wrap bottom layer 46 at least partially or fully enclose the absorbent layer 30, providing for dry and wet immobilization of the absorbent layer. Additionally, the absorbent layer may be further immobilized within the core wrap by an hot melt adhesive. The adhesive may for example be applied on the inner side of at least one of the top layer 45 and the bottom layer 46 core wrap. For airfelt-free core having an absorbent material consisting of SAP particles, it is also known to immobilize the SAP particles using a micro-fibrous glue applied on the particles and immobilizing these to the inner side of the core wrap layers.

The absorbent layer 30 comprises or consist of superabsorbent polymer particles. The superabsorbent particles may be mixed with cellulose fibers, or alternatively the absorbent material may be free of cellulose fibers. The absorbent layer may comprise, by weight of the absorbent layer, at least 30%, or at least 40%, or at least 50%, or at least 60%, or at least 70%, or at least 80%, or at least 90% of superabsorbent polymer particles. The absorbent layer may comprise, by weight of the absorbent layer, less than 50% of cellulose fibers, in particular less than 25%, or less than 20%, or less than 15% or less than 10 weight% of cellulose, or less than 5% by weight of cellulose fibers, and may be free of cellulose fibers.

Various absorbent core designs comprising high amounts of SAP have been proposed in the past, see for example in US5,599,335 (Goldman), EP1,447,066 (Busam), WO95/11652 (Tanzer), US2008/0312622A1 (Hundorf), WO2012/052172 (Van Malderen). In particular, the SAP printing technology as disclosed in US7,838,722 (Blessing), US9,072,634 and US8,206,533 (both to Hundorf et al.) may be used. The present disclosure however is not limited to a particular type of absorbent core. The absorbent core may also comprise one or more glues such as an auxiliary glue applied between the internal surface of one (or both) of the core wrap layers and the absorbent material to reduce leakage of SAP outside the core wrap. A micro-fibrous adhesive net may also be used in air-felt free cores as described in the above Hundorf references. These glues are not represented in the Figures for simplicity. Other core constructions comprising a high loft nonwoven substrate, such as a carded nonwoven layer, having a porous structure into which SAP particles have been deposited, may also be used in present disclosure.

When the absorbent layer is free of cellulose fibers, the absorbent layer may consist of the superabsorbent polymer particles. The resulting absorbent layer has a reduced thickness in the dry state compared to conventional absorbent cores including cellulosic fibers. The reduced thickness helps to improve the fit and comfort of the absorbent article for the wearer.

The absorbent layer 30 defines a deposition area, when considered in the plane of the absorbent article as shown in Fig. 1, having a pre-determined shape. The absorbent layer 30 may have any shape, in particular a rectangular shape as illustrated in Fig. 1, but other shapes are common such as dog-bone or sand-hour shaped having a tapering in the crotch region of the article.

The absorbent layer 30 may define one or more channel(s) 26, where substantially no absorbent material is present, apart from possible accidental discrete contamination. These absorbent material-free channels preferably do not extend to any of the side of the absorbent material layer. The channels may thus be completely surrounded by absorbent material. The channels are typically elongated in the longitudinal direction, having a length of from 20% and 80%, or from 20% to 70%, or from 30% to 60%, by total longitudinal dimension of the absorbent material layer 30. The channel may be straight, curved, or combinations thereof. The one or more channels are typically symmetrically disposed relative to the longitudinal axis When a pair of channels is present, these may be discrete, i.e. not connected with another, as illustrated in Fig. 1, or alternatively the channels 26 may be connected at one or both their extremities to form a U or O shape, or , H, T, X, V, >< shapes. Examples of such absorbent material-free channels are disclosed in further details in WO2012170778A1, WO2012170781 (Kreuzer et al.).

The core wrap top layer 45 and core wrap bottom layer 46 may be bonded to each other through at least a portion of the length of the channel. This bond provides for structural integrity of the channels in dry and wet state. Any known bonding techniques known in the art may be used to provide for this bond. The channel bonding of the core wrap layers may be selected from adhesive bonding, thermo bonding, mechanical bonding, ultrasonic bonding, or any combinations thereof. An adhesive may be for example applied in the areas of the channels on the inner side of the top side and/or the inner side of the bottom side of the core wrap, typically by slot glue application or any other means, followed by the application of pressure in the areas of the channels to provide a good adhesive bonding in these areas. Exemplary patent disclosures of such adhesive bonding processes can be found for an airfelt or airfelt-free absorbent cores in WO2012/170798Al (Jackels et al.), EP2,905,000 (Jackels et al.), WO2016/149251A1 (Stiehl et al.) and EP2,905,001 (Armstrong-Ostle et al.).

Other bonding such as thermo bonding, mechanical bonding, ultrasonic bonding can also be used as additional bonding or as an alternative bonding. For example, an adhesive bonding may be reinforced by a thermo bonding, mechanical bonding or ultra-sonic bonding. Such thermo, mechanical or ultrasonic bonding can be applied on the channels through the external sides of the core wrap substrates.

Typically, the bonds may generally have the same outline and shape as the channels 26 in which they are contained but may be slightly smaller to allow for a safety margin (e.g. by a few mm) as some deviations from the optimal registration may happen during high-speed process. The absorbent material free channels may also be not bonded or have one or more section which is bonded and one or more section that is not bonded.

Suitable SAP may be any water-insoluble, water-swellable polymers capable of absorbing large quantities of fluids, as is known in the art. "Superabsorbent polymer" or "SAP" refers herein to absorbent materials, typically cross-linked polymeric materials, that can absorb at least 10 times their weight of an aqueous 0.9% saline solution as measured using the Centrifuge Retention Capacity (CRC) test as indicated in EDANA method NWSP 241.0.R2 (19). The SAP may in particular have a CRC value of at least 20 g/g, or more than 24 g/g, or of from 20 g/g to 50 g/g, or from 20 g/g to 40 g/g, or 24 g/g to 35 g/g. "Superabsorbent polymer particles", as used herein, refers to a superabsorbent polymer material which is in particulate form so as to be flowable in the dry state.

### Acquisition layer 52

As indicated previously, absorbent articles typically comprise an acquisition layer 52 directly underneath the topsheet to quickly acquire urine or other bodily fluid away from the topsheet and the skin of the wearer. The acquisition is an intermediate layer between the topsheet and the absorbent core which comprises the absorbent material to lock the insulting fluid within the article.

The absorbent article can optionally a secondary acquisition layer, also denominated as distribution layer 54 between a primary acquisition layer 52 and the absorbent core 28. This is known in the art when the absorbent material of the absorbent core is free of cellulose fibers, where the distribution layer is typically an airlaid layer consisting of cross-linked cellulose fibers. More generally, if a secondary acquisition layer is present, this secondary layer may be the acquisition layer made of the same bi-functional material as the cushion layer. In this case, where a primary and a secondary acquisition layers are present in the article, the bi-functional material acquisition layer of the invention may be either of these (or even both if the acquisition layer was made a double layer).

### Cushion layer 60, 60'

As indicated above, absorbent articles comprising a high proportion of SAP particles in the absorbent layer may benefit from having a cushion layer that feels soft to the touch on the garment-facing side of the article. Even conventional cores that comprises cellulose fibers may benefit from additional cushiness on the garment-facing side of the article. The cushion layer is usually a lofty layer having good pressure recovery properties, as will be detailed in the next sections.

The cushion layer may be advantageously disposed between the core wrap bottom layer 46 and the liquid-impermeable backsheet 25, as illustrated in Figs. 1-2. This construction is also the simplest to make, as it does not require making large changes to existing absorbent core making process. As is disclosed in the process section below, the cushion layer may be being between the absorbent core and the backsheet in the article converting lines.

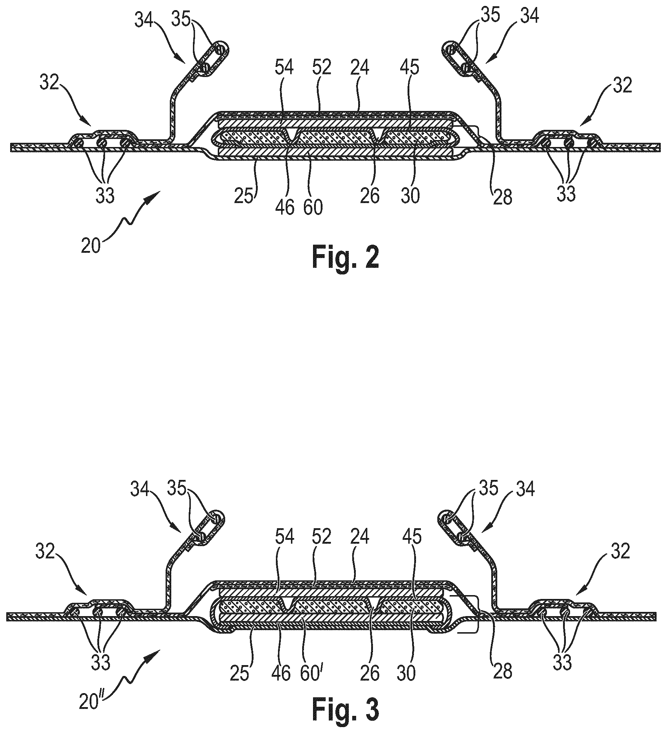

Alternatively, as illustrated in Fig. 3, the cushion layer 60' may be disposed within the core wrap between the absorbent layer 30 and the core wrap bottom layer 46.

### Bi-functional material

According to the present invention, the inventors have found that a bi-functional material can serve as an acquisition layer disposed between the topsheet and the absorbent layer and as a cushion layer disposed between the absorbent layer and the backsheet, allowing a more economic process for making the articles than if the layers were made from different materials.

The bi-functional material used in the present invention should combine liquid handling properties required for fluid acquisition and tactile properties required for softness and cushiness. These properties can be measured by different parameters and the associated test methods as indicated below and in the "TEST METHODS SECTION" below.

The cushion layer and the acquisition layer may have the same or different width, but preferably have the same length as measured in the longitudinal direction so that they can obtained by continuously slitting the same roll of bi-functional material, as illustrated in the process below.

The bi-functional material of the invention may typically be a nonwoven, as these materials are known for use in absorbent article and can be conveniently provided as rolls of material that can be continuously slit in two sub-webs to form the cushion layer and the acquisition layer in a process of the invention. Examples of bi-functional materials that can be used in the present invention are indicated in the examples section below. Resin bonded carded and through air bonded carded nonwovens may be particularly suitable, but are not exclusive. Other suitable materials are for example spunlace nonwovens.

The cushion layer and the acquisition layer are made from the same bi-functional material, meaning these two different layers of the articles have the same intrinsic properties such as basis weight, density, caliper as well as liquid handling capacity, which are thus balanced for both usages.

The basis weight of the bi-functional material may typically be in the range of from 14 g/m² to 100 g/m², in particular of from 20 g/m² to 80 g/m², or from 30 g/m² to 60 g/m², and for example about 40 g/m². The basis weight of the bi-functional material is typically homogeneous throughout the length and width of the bi-functional layer(i.e. in the longitudinal and transverse direction). The basis weight of a material is typically provided by the supplier, but can also be calculated by dividing the weight of a sample of the bi-functional material by its surface.

Regarding the cushion properties, while it is generally true that lofty nonwoven material may be cushiony, lofty material may also generally increase the thickness of the article, which can reduce wearer comfort. Also, increased bulk is generally not desirable, especially between the wearer's legs. Therefore, a good compromise is for the bi-functional material as cushion layer to have a Caliper @2.1 kPa of at least 0.3 mm, for example from 0.4 mm to 2 mm or from 0.5 mm and 1 mm as measured according to the Caliper Measurement Method described herein.

The density of the bi-functional material may typically be in the range of from 0.5 g/cm³ to 3 g/cm³ as measured at 2.1 kPa pressure according to the Caliper Measurement Method described herein.

Advantageously, the bi-functional material also has cushiony properties that can be directly measured as Z-Compliance index of at least 10 mm³/N , more preferably between 15 mm³/N and 60 mm³/N, even more preferably between 25 mm³/N and 50 mm³/N. The Recovery from compliance index of at least 40%, preferably at least 60%, more preferably of at least 70% and up to 100%, both parameter being measured according to the Z-Compliance Index and Percent Recovery Measurement Method disclosed herein.

The bi-functional material also has good liquid acquisition properties so that it can serve as acquisition layer between the topsheet and the absorbent core. The bi-functional may comprise a surfactant to increase its hydrophilicity. The surfactant may be introduced in the bi-functional material according to any of the methods known in the art. The surfactant may be sprayed or otherwise applied on the surface of the bi-functional material, or integrated in the polymer matrix during the making of the fibers, as is known in the art. The bi-functional may also or instead comprise inherently hydrophilic fibers such as cellulose fibers, which may or may not be mixed with synthetic fibers, as is known for example in spunlace nonwoven material.

Advantageously, the bi-functional material has relatively high permeability. Permeability generally refers to the quality of a porous material that causes it to allow liquids or gases to pass through it and, as such, is generally determined from the mass flow rate of a given fluid through it. The permeability of a material is related to the material's ability to quickly acquire and transport a liquid within the structure, both of which are key features of an absorbent article. Accordingly, measuring permeability is one metric by which a material's suitability for use in absorbent articles may be assessed. The permeability of the bi-functional material can be measured by the In-Plane Radial Permeability (IPRP) Test Method disclosed further below, expressed in SFC (1 SFC = 10⁶ cm/Pa sec).

The bifunctional material may in particular have IPRP of at least 4,000 SFC preferably between 5,000 and 30,000 more preferably between 6,000 and 20,000 SFC.

### Process for making the article and slitting device

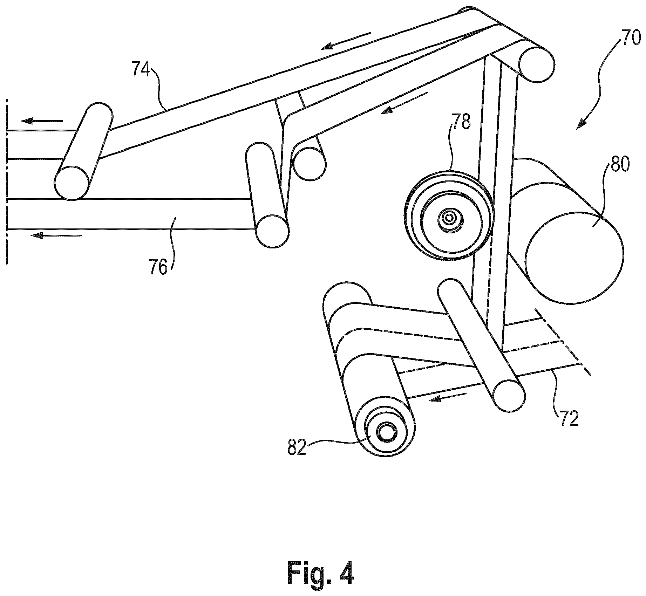

Having described an exemplary absorbent article of the invention with reference to Figs. 1-3, an industrial process to make these articles maybe better described by starting with the schematic representation of a slitting device 70 shown in Fig. 4, which is part of the converting line implementing the process of the invention. As illustrated in Fig. 4, a suitable bi-functional material web 72 is unwind from a roll of material (not shown) and is continuously fed into the slitting device 70 as input material. Herein, the wording "material" refers to the web or component of the article being provided and converted on the converting line, while the wording "layer" is used with reference to the individualized component which has been integrated in the chassis and ultimately having been made into the article following superposing, cutting and assembling the other layers and components of the invention.

The slitting device 70 as shown in Fig. 4 comprises a circular blade 78 and anvil 80 system for continuously slitting the bi-functional material web along its middle or at another transversal point. A driving roll 82 may be placed before the blade and anvil system to keep the web well-tensed during the slitting step and to help separate the slitted nonwoven web into first and second sub-webs.

The position of the blade 78 and anvil 80 is of course interchangeable. The output of the slitting device 70 is a first sub-web 74 and second sub-web 76, which can then be used as acquisition material and cushion material respectively, or vice versa. The width of the first sub-web and second sub-web is represented equal in the present illustration, but the widths of the sub-webs may also be different if it is preferred to have an acquisition layer and cushion layer of different widths. The position of the blade can be adjusted in cross-direction in case the design of the article rather require a wider acquisition layer relative to the cushion layer, or vice versa. However it may be preferred to have the materials of equal width to provide for balanced acquisition and cushion properties over the same area of the article.

Advantageously one of the sub-webs 74 is positioned so that it exits the slitting device already generally aligned with the other materials and the converting line so that the first sub-web does not need realigning. The second sub-web 76 then of course exits the slitting device in an offset position in cross-direction respective to the first sub-web 74 and the converting line so that it needs to be realigned with the other materials.

This realignment may be advantageously done by using two angled bars (not shown) to realign the second sub-web 76 with the first sub-web 74 and the other materials in the converting line. The two angled bars may be each angled at about 45 degrees relative to the machine direction of the nonwoven web. The process of the invention is of course not limited to a particular slitting device configuration, any slitting device system and placement as known in the art may be used in the present invention. A slitting device is for example disclosed in US2013/0172166A1 (Erguen et al.).

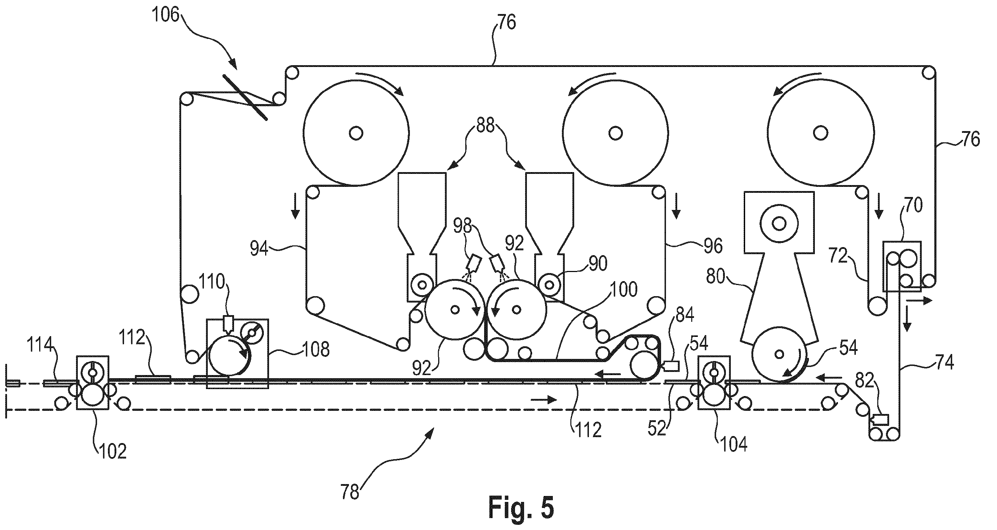

The first sub-web 74 and second sub-web 76 exiting the slitting device are then converted with other materials that are provided to the converting line 84, an embodiment of which is represented schematically in Figs. 5-7, for making the absorbent articles 20.

### Converting lines

Fig. 5 schematically shows a portion 78 of a converting line for making an absorbent system 114 for an absorbent article as illustrated in Figs 1-2 wherein the cushion layer 60 is disposed between the absorbent core and the backsheet. The absorbent system encompasses the layers between the topsheet and backsheet, including acquisition layer 52, absorbent core 28 and cushion layer 60, and optionally a distribution layer 54. The process illustrated in Fig. 5 includes a specific core making process for making core by SAP printing technology as discussed above, however any absorbent cores making process may be used, in particular a standard airfelt making core process comprising mixing SAP particles and cellulose fibers in a chute and laying them on a drum, as is known in the art.

Referring to Fig. 5, the bi-functional layer in the form of a web 72 is provided by continuously unwinding a roll bi-functional material and feeding the bi-functional material 72 to the slitting device 70 as disclosed above with reference to Fig. 4.

A first sub-web 74 exiting the slitting device may be used to form as the acquisition layer 52. In the exemplary process of Fig. 5, the first sub-web 74 is combined with an airlaid distribution layer 54 made in the laydown unit 80, as is known in the art. The distribution layer may consist of cross-linked cellulose fibers as indicated above. The distribution layer may or may not comprise channels as desired and set by the shape of the airlaid mold used. When one or more channels are present in the distribution layer 54, and these may be registered with the channels 26 in the absorbent core 28, if present. The distribution layer is typically shorter in the longitudinal and/or transversal direction than the acquisition layer, so that the acquisition material 74 can be used as a substrate for the distribution layer 54 to be deposited thereon. The distribution layer 54 is deposited in the laydown unit already in discrete form on the first sub-web / acquisition material. A contact glue applicator 82 may be used to apply an adhesive on the first sub-web 74 before airlaying the distribution layer 54 to provide for adhesion between the first sub-web / acquisition material and the distribution layer.

The first sub-web 74, optionally comprising a distribution layer 54, is transferred via conveying rolls to a cut and slip unit 84 to individualize the first sub-web of bi-functional material into discrete acquisition layers. In this example, the sub-web 74 is cut in the gap between two consecutive distribution layers 54. The acquisition layer / distribution layer laminate is then combined with an absorbent core material 100 in a next step, which may be provided as exemplified in the next paragraph.

The converting line may use any type of absorbent core material to make the absorbent assembly. Fig. 5 illustrates an exemplary air-felt free core making unit 88 comprising two sub-units each having a printing roll 90 cooperating with a lay-on drum 92 for depositing SAP particles on continuously unwound nonwoven substrates 92, 94. These core substrates are typically SMS materials used to form the core wrap top layer and a core wrap bottom layer respectively. The SAP particles may be immobilized by a microfibrous glue applied from spray applicator 98 on the SAP once the SAP has been deposited on the substrate. The two half layers each comprising a substrate and a SAP layer are combined in face-to-face relationship to form a continuous absorbent core material 100 exiting the absorbent core making unit 88. One or more absorbent material free channel 26 may be formed in the absorbent core, as disclosed in the art referred above.

Of course this is only one example of core making process, any type of absorbent core including airfelt containing core or composite core comprising a high loft layer within which SAP particles are distributed may be provided to the converting line. The absorbent core may also be a pre-made core made in another line and supplied to the converting line as a roll or as a core bale, which can be described as a continuous band of material that is stored flat in a quadratic container, as is known in the art. The absorbent core material 100 may be applied with an adhesive from an adhesive contact applicator 104 before combining with the acquisition layer / distribution layer laminate 52, 54, the distribution layer 54 being directly in contact with the absorbent core material 100.

Turning now to the second sub-web 76, as disclosed in the process of Fig. 5, this sub-web can be converted into the cushion layer 60 of the absorbent article. The second sub-web 76 may be optionally flipped as schematically represented by the flipping unit 106 so that the same side of the first and second sub-web faces towards the absorbent core. The second sub-web 76 may be applied with a contact adhesive via contact applicator 110 before being fed in a cut-and slip unit 108 to individualize the sub-web 76 into individual cushion layer 60. The individualized cushion layers 60 are then combined with the side of the absorbent core material 100 opposed to the side comprising the already formed the acquisition layer 52 and the opinion distribution layer 54. The thus formed absorbent system laminate 112 is then brought to the cut and slip unit 102 where the absorbent core material is cut transversally in CD to form individualized absorbent systems 114.

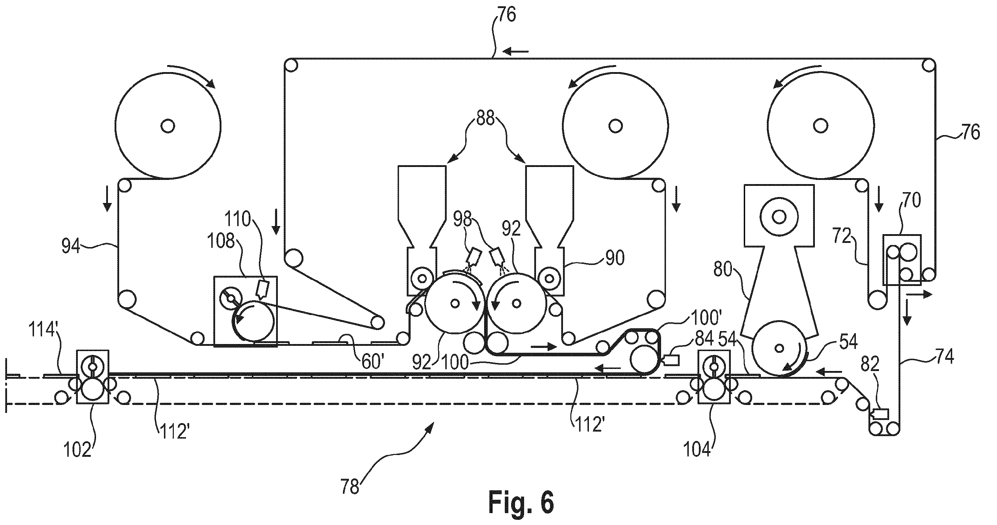

Fig. 6 shows an alternative process that can be used to make absorbent articles as shown in Fig. 3 where the cushion layer 60' is integrated in the absorbent core 28. In this process, the second sub-web material 76 can be applied with an adhesive via the contact applicator 110 before being fed to cut and slip unit 108 as described before, with the difference what the cushion layers 60' formed are deposited on the inner surface of the core wrap material 94, so that the absorbent material is deposited on top of the laminate formed by the cushion layer and the core wrap substrate in one of the core making subunit 88. The absorbent assembly laminate 112' is further individualized in absorbent assembly 114' as indicated previously.

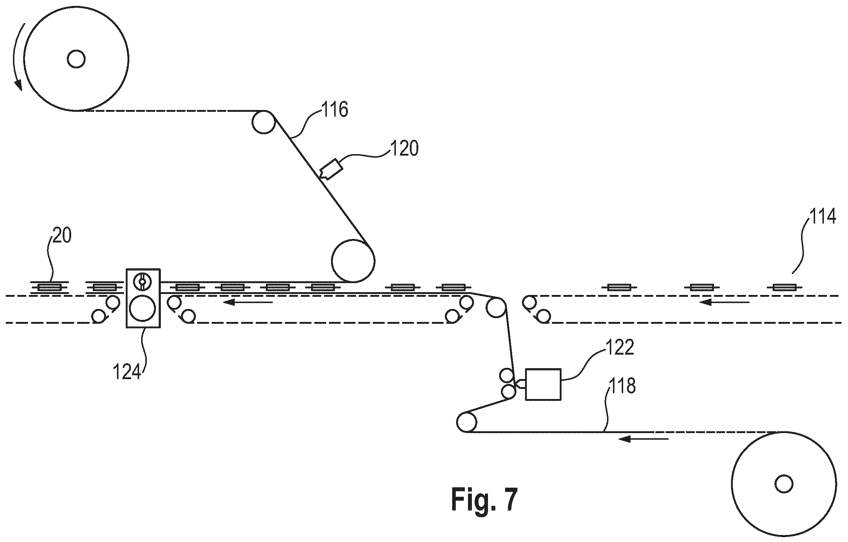

The steps of finishing the absorbent articles by combining the absorbent system 114 with a backsheet material 116 and a topsheet material 118 is schematically described in Fig. 7. The backsheet material 116 is provided by unrolling a continuous roll of backsheet material. An adhesive may be applied on the backsheet using glue applicator 120, typically as a series of glue spirals. From another side of the converting line, the topsheet material 118 may be similarly continuously provided from a roll of material, as is known in the art. Back and front ears may be attached to the topsheet material before combining the topsheet material with the rest of the article in a step, which is not shown, but is usual in the art. Likewise leg cuffs and their elastics (not represented) are also continuously provided and disposed longitudinally on each side of the absorbent system. The backsheet material, the layers forming the absorbent systems which have already been individualized and the topsheet material are continuously combined typically by a combination of adhesives and fusion bonds. Other optional components of the article such as a back waist cuff and front waist band, for example as disclosed in WO2020/242715 (Raycheck et al.), an urine indicator, for example as disclosed in WO2015/095514 (Joseph), etc.... can of course be combined with the other components of the absorbent article as is known in the art. The core bag formed by the core wrap top layer and core wrap bottom layer may overlap with the back waist cuff or front waist bond, or the core bag may be spaced in the longitudinal direction from the back waist cuff and/or front waist band. A final cross-direction knife cut is provided to individualize the absorbent articles in the cut and slip unit 124. The individualized articles are then folded and packaged in a filling and packing station as is known in the art (these steps not being represented in the Figures).

The present process provides a cost-effective way of making the articles of the invention in comparison to other processes. In particular, the continuous splitting of the bi-functional material into two sub-webs eliminates the need for an additional unwinder, reduces waste due to material handling (caused by the need for splicing when changing rolls of materials), and reduces line layout floor space in a manufacturing plant. In comparison, a splitting device as used in the present process is comparatively low cost and takes up little space.

### Packages

A plurality of articles according to the invention may be comprised in a package for transport and sale. At least 50% of the articles, and preferably all the articles, in the package may be according to the invention. The absorbent articles may be folded and packaged as is known in the art. The package may be for example a plastic bag or a cardboard box. Diapers may typically bi-folded along the transversal axis and the ears folded inwardly before being packaged. The absorbent articles may be packed under compression so as to reduce the size of the packages, while still providing an adequate number of absorbent articles per package. By packaging the absorbent articles under compression, caregivers can easily handle and store the packages, while also providing distribution and inventory savings to manufacturers owing to the size of the packages.

The absorbent articles may thus be packaged compressed at an In-Bag Compression Rate of at least 10%, in particular of from 10% to 50%, in particular from 20% to 40%. The "In-Bag Compression Rate" as used herein is one minus the height of a stack of 10 folded articles measured while under compression within a bag ("In-Bag Stack Height") divided by the height of a stack of 10 folded articles of the same type before compression, multiplied by 100; i.e. (1-In-Bag Stack Height/stack height before compression) * 100, reported as a percentage. Of course, the stack in the bag does not need to have exactly 10 articles, rather the value measured for the height of stack of article in the package is divided by the number of articles in the stack and then multiplied by 10. The method used to measure the In-Bag Stack Height is described in further details in the Test Procedures. The articles before compression are sampled from the production line between the folding unit and the stack packing unit. The stack height before compression is measured by taking 10 articles before compression and packing and measuring their stack height as indicated for the IBSH.

Absorbent article packages of the present disclosure may in particular have an In-Bag Stack Height of less than 110 mm, less than 105 mm, less than 100 mm, less than 95 mm, less than 90 mm, specifically reciting all 0.1 mm increments within the specified ranges and all ranges formed therein or thereby, according to the In-Bag Stack Height Test described herein. For each of the values indicated in the previous sentence, it may be desirable to have an In-Bag Stack Height of greater than 60, or greater than 70 mm, or greater than 75 mm, or greater than 80 mm. Alternatively, packages of the absorbent articles of the present disclosure may have an In-Bag Stack Height of from 60 mm to 110 mm, from 65 mm to 110 mm, from 70 mm to 110 mm, from 75 mm to 105 mm, or from 80 mm to 100 mm, specifically reciting all 0.1 mm increments within the specified ranges and all ranges formed therein or thereby, according to the In-Back Stack Height Test described herein.

### Bio-sourced materials

Components of the disposable absorbent article (i.e., diaper, pant, sanitary napkin, pantiliner, etc.) of the present invention can at least partially be comprised of bio-sourced content as described in US 2007/0219521A1 Hird et al published on September 20, 2007, US 2011/0139658A1 Hird et al published on June 16, 2011, US 2011/0139657A1 Hird et al published on June 16, 2011, US 2011/0153012A1 Hird et al published on June 23, 2011, US 2011/0139662A1 Hird et al published on June 16, 2011, and US 2011/0139659A1 Hird et al published on June 16, 2011. These components include, but are not limited to, topsheet nonwovens, backsheet films, backsheet nonwovens, , barrier leg cuff nonwovens, superabsorbent layer, upper and lower substrate layer, adhesives, fastener hooks, and fastener landing zone nonwovens and film based.

The disposable absorbent article component may comprise a bio-based content value from about 10% to about 100% using ASTM D6866-10, method B, in another embodiment, from about 25% to about 75%, and in yet another embodiment, from about 50% to about 60% using ASTM D6866-10, method B.

In order to apply the methodology of ASTM D6866-10 to determine the bio-based content of any disposable absorbent article component, a representative sample of the disposable absorbent article component must be obtained for testing. Thereto, the disposable absorbent article component may be ground into particulates less than about 20 mesh using known grinding methods (e.g., Wiley^{®} mill), and a representative sample of suitable mass taken from the randomly mixed particles.

### Examples

The following are examples of bi-functional materials that may be used in the present invention.

| | **Example 1: 43 gsm resin bound nonwoven** | **Example 2: 33 gsm resin bonded nonwoven** | **Example 3 40 gsm Air Through bonded nonwoven** |
|---|---|---|---|
| Fiber Composition: | 40:60 ratio of 6/9 den PET (66-73wt%) with 30% target SBS Resin Binder | 100% 6 den PET fibers w/ 30% target SBS Resin Binder | 60% 2 den semi durable hydrophilic CoPET /PET fibers and 40% 4 den durable hydrophilic PE/PET |
| Compliance Index (mm³/N | 46 | 37 | 13 |
| Recovery from compliance index | 78 % | 75 % | 87 % |
| Caliper @2.1Kpa | 0.45 mm | 0.56 mm | 0.37 mm |
| IPRP (SFC) | 8683 | 14093 | 1478 |

### Test Methods

### Z-Compliance Index and Percent Recovery Measurement Method

*Principle:* This method measures the ability of a nonwoven web to be compressed in z-direction under applied pressure and then to recover to its original caliper after removing said applied pressure.

*Setup:* A vertically oriented electronic caliper tester having a precision of at least 0.01 mm with a 40 mm diameter circular foot may be used. The pressure exerted by the foot on the specimen is adjustable via the addition of pre-selected weights. Measurements are made at 0.85 ± 0.05 kPa and 15.4 ± 0.1kPa.

*Procedure:* Measurements are performed at 23°C ± 2°C and 50% ± 2% RH. All samples should be kept at least 24 hours in these conditions to equilibrate before conducting the tests, unless indicated otherwise. If possible, measurements are made on the lower ADS before it is integrated in an absorbent article. If this is not possible, care should be exerted when excising the sample to not impart any contamination or distortion to the test sample layer during the removal the material from other layers (using cryogenic spray, such as Cyto-Freeze, Control Company, Houston, Texas, if needed). The specimen is cut from to a square sample with a width of about 80 mm (or alternatively in case the material is not available in the suitable size in a material specimen with a width of about 50 mm).

The square sample specimen is positioned centered under the caliper foot and the caliper at 0.85 ± 0.05 kPa (P1) is measured and recorded to the nearest 0.01 mm (C1). Without removing the sample from the equipment, the pressure is increased to 15.4 ± 0.1 kPa (P2) and the caliper measured and recorded to the nearest 0.01 mm (C2). The pressure may be increased by adding a suitable weight on the caliper foot. Again without moving the sample, the exerted pressure is reduced back to 0.85 ± 0.05 kPa (for example by removing the extra weight) and the caliper measured a third time (C3) and recorded to the nearest 0.01mm.

For the specimen being measured, the compliance index is defined as: $Z - compliance index = \left(C1-C2\right) / \left(P2-P1\right)$ and is recorded to the nearest 0.1 mm³/N.

The recovery is calculated as: $recovery = C 3 / C 1 * 100 %$ expressed in percent and recorded to the nearest 0.1%.

The procedure above is conducted on five like specimens of the same nonwoven. The arithmetic mean of the compliance index values among the five specimens is calculated and reported to the nearest 0.1 mm³/N as the Compliance Index. The arithmetic mean of percent recovery values among the five specimens is calculated and reported to the nearest 0.1 % as the Percent Recovery.

### Caliper Test Method

The Caliper of the bi-functional material is determined using this Caliper Test Method. In the Caliper Test Method, two flat, parallel surfaces are used to apply unidirectional pressure to both sides of a substrate specimen, and the resulting separation between the parallel surfaces is measured. All measurements are performed in a laboratory maintained at 23 ± 2 °C and 50 ± 2 % relative humidity and test specimens are conditioned in this environment for at least 2 hours prior to testing.

Two parallel circular surfaces of 5.6 cm diameter are oriented horizontally. If possible, measurements are made on the intermediate layer before it is integrated in an absorbent article. If this is not possible, care should be exerted when excising the intermediate layer from the product not to impart any contamination or distortion to the test sample layer during the removal of the material from other layers (using cryogenic spray, such as Cyto-Freeze, Control Company, Houston, Texas, if needed). Five equivalent rectangular specimens are taken from the bi-functional material such that each specimen center corresponds to the center of the bi-functional material and such that the length and width of each specimen is greater than 5.6 cm. A specimen is then placed between the two parallel circular surfaces so that it completely covers each of the parallel surface and such that the center of the intermediate layer specimen is matching with the center of the parallel circular surfaces. The caliper of the bi-functional material is measured on the raw material before it is converted into an article.

The parallel surfaces are then brought together at a rate of 3.0 ± 1.0 mm/s until a pressure of 0.3 psi (2.1 kPa) is achieved, and the separation between the plates is measured and recorded to the nearest 0.01 mm within 2 seconds. The arithmetic mean of the plate separation of the 5 individual replicate specimens is calculated and reported as the Caliper of the substrate under 2.1 kPa in units of millimeters (mm) to the nearest 0.01 mm.

One suitable example of apparatus for use in the Caliper Method is a Mitutoyo Digimatic Series 543 ID-C digital indicator (Mitutoyo America Corp., Aurora, Illinois, USA), or equivalent, fitted with a circular flat "foot" at the end of the moving shaft of the indicator gauge. The indicator is mounted on a horizontal granite base such that the shaft of the indicator gauge is oriented vertically and the plane of the circular foot is parallel to the granite base. The circular foot is sized and weighted such that the gravitational force associated with the mass of the foot and the indicator shaft together divided by the area of the circular foot constitutes 2.1kPa (0.3 psi) of downward pressure from the circular foot on the granite base. Specimens at least as large as the circular foot are analyzed between the circular foot and granite base.

### In-Plane Radial Permeability (IPRP) Test Method

Permeability generally refers to the quality of a porous material that causes it to allow liquids or gases to pass through it and, as such, is generally determined from the mass flow rate of a given fluid through it. The permeability of an absorbent structure is related to the material's ability to quickly acquire and transport a liquid within the structure, both of which are key features of an absorbent article. Accordingly, measuring permeability is one metric by which a material's suitability for use in absorbent articles may be assessed.

Permeability generally refers to the quality of a porous material that causes it to allow liquids or gases to pass through it and, as such, is generally determined from the mass flow rate of a given fluid through it. The permeability of an absorbent structure is related to the material's ability to quickly acquire and transport a liquid within the structure, both of which are key features of an absorbent article. Accordingly, measuring permeability is one metric by which a material's suitability for use in absorbent articles may be assessed.

The following test is suitable for measurement of the In-Plane Radial Permeability (IPRP) of a porous material. The quantity of a saline solution (0.9% NaCl) flowing radially through an annular sample of the material under constant pressure is measured as a function of time.

Testing is performed at 23°C ± 2C° and a relative humidity 50% ± 5%. All samples are conditioned in this environment for twenty-four (24) hours before testing.

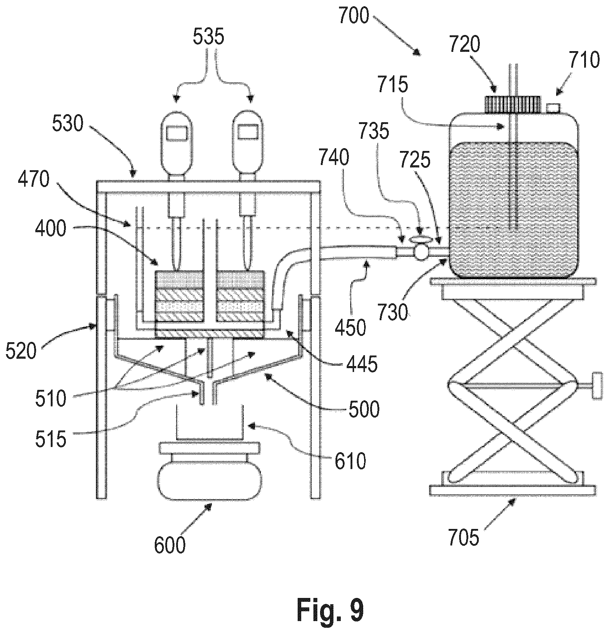

The apparatus -or parts thereof- as described below are shown in Figures 9 and 10.

The IPRP sample holder 400 is shown in FIG. 9 and comprises a cylindrical bottom plate 405, top plate 410, and cylindrical stainless-steel weight 415.

Top plate 410 comprises an annular base plate 420, which is 9 mm thick with an outer diameter of 70 mm and a tube 425 of 150 mm length fixed at the center thereof. The tube 425 has an outer diameter of 15.8 mm and an inner diameter of 12 mm. The tube is adhesively fixed into a circular 16 mm hole in the center of the base plate 420 such that the lower edge of the tube is flush with the lower surface of the base plate, as depicted in FIG. 21. The bottom plate 405 and top plate 410 are fabricated from Lexan^{®} or equivalent. The stainless-steel weight 415 has an outer diameter of 70 mm and an inner diameter of 15.9 mm so that the weight is a close sliding fit 20 on tube 425. The thickness of the stainless-steel weight 415 is approximately 22 mm and is adjusted so that the total weight of the top plate 410 and the stainless steel weight 415 is 687g ± 1g to provide 2.0 kPa of confining pressure during the measurement.

Bottom plate 405 is approximately 25 mm thick and has two registration grooves 430 cut into the lower surface of the plate such that each groove spans the diameter of the bottom plate and the grooves are perpendicular to each other. Each groove is 1.5 mm wide and 2 mm deep. Bottom plate 405 has a horizontal hole 435 which spans the diameter of the plate. The horizontal hole 435 has a diameter of 8 mm and its central axis is 15 mm below the upper surface of bottom plate 405. Bottom plate 405 also has a central vertical hole 440 which has a diameter of 8 mm and is 10 mm deep. The central hole 440 connects to the horizontal hole 435 to form a T-shaped cavity in the bottom plate 405. The outer portions of the horizontal hole 435 are threaded to accommodate pipe elbows 445 which are attached to the bottom plate 405 in a watertight fashion. One elbow is connected to a vertical transparent tube 460 with a total height of 175 mm measured from the bottom of bottom plate 405 (including elbow 445) and an internal diameter of 6 mm. The tube 460 is scribed with a suitable mark 470 at a height of 100 mm above the upper surface of the bottom plate 420. This is the reference for the fluid level to be maintained during the measurement. The other elbow 445 is connected to the fluid delivery reservoir 700 (described below) via a flexible tube.

A suitable fluid delivery reservoir 700 is shown in FIG. 10. Reservoir 700 is situated on a suitable laboratory jack 705 and has an air-tight stoppered opening 710 to facilitate filling of the reservoir with fluid. An open-ended glass tube 715 having an inner diameter of 10 mm extends through a port 720 in the top of the reservoir such that there is an airtight seal between the outside of the tube and the reservoir. Reservoir 700 is provided with an L-shaped delivery tube 725 having an inlet 730 that is below the surface of the fluid in the reservoir, a stopcock 735, and an outlet 10 740. The outlet 740 is connected to elbow 445 via flexible plastic tubing 450 (e.g., Tygon^{®}). The internal diameter of the delivery tube 725, stopcock 735, and flexible plastic tubing 450 enable fluid delivery to the IPRP sample holder 400 at a high enough flow rate to maintain the level of fluid in tube 460 at the scribed mark 470 at all times during the measurement. The reservoir 700 has a capacity of approximately 6 liters, although larger reservoirs may be required depending on the sample thickness and permeability. Other fluid delivery systems may be employed provided that they are able to deliver the fluid to the sample holder 400 and maintain the level of fluid in tube 460 at the scribed mark 470 for the duration of the measurement.

The IPRP catchment funnel 500 is shown in Fig. 10 and comprises an outer housing 505 with an internal diameter at the upper edge of the funnel of approximately 125 mm. Funnel 500 is constructed such that liquid falling into the funnel drains rapidly and freely from spout 515. A stand with horizontal flange 520 around the funnel 500 facilitates mounting the funnel in a horizontal position. Two integral vertical internal ribs 510 span the internal diameter of the funnel and are perpendicular to each other. Each rib 510 is 1.5 mm wide and the top surfaces of the ribs lie in a horizontal plane. The funnel housing 500 and ribs 510 are fabricated from a suitably rigid material such as Lexan^{®} or equivalent in order to support sample holder 400. To facilitate loading of the sample it is advantageous for the height of the ribs to be sufficient to allow the upper surface of the bottom plate 405 to lie above the funnel flange 520 when the bottom plate 405 is located on ribs 510. A bridge 530 is attached to flange 520 in order to mount two digital calipers 535 to measure the relative height of the stainless-steel weight 415. The digital calipers 535 have a resolution of ± 0.01 mm over a range of 25 mm. A suitable digital caliper is a Mitutoyo model 543- 492B or equivalent. Each caliper is interfaced with a computer to allow height readings to be recorded periodically and stored electronically on the computer. Bridge 530 has a circular hole 22 mm in diameter to accommodate tube 425 without the tube touching the bridge.

Funnel 500 is mounted over an electronic balance 600, as shown in FIG. 10. The balance has a resolution of ± 0.01 g and a capacity of at least 1000 g. The balance 600 is also interfaced with a computer to allow the balance reading to be recorded periodically and stored electronically on the computer. A suitable balance is Mettler-Toledo model MS6002S or equivalent. A collection container 610 is situated on the balance pan so that liquid draining from the funnel spout 515 falls directly into the container 610.

The funnel 500 is mounted so that the upper surfaces of ribs 510 lie in a horizontal plane. Balance 600 and container 610 are positioned under the funnel 500 so that liquid draining from the funnel spout 515 falls directly into the container 610. The IPRP sample holder 400 is situated centrally in the funnel 500 with the ribs 510 located in grooves 430. The upper surface of the bottom plate 405 must be perfectly flat and level. The top plate 410 is aligned with and rests on the bottom plate 405. The stainless-steel weight 415 surrounds the tube 425 and rests on the top plate 410. Tube 425 extends vertically through the central hole in the bridge 530. Both calipers 535 are mounted firmly to the bridge 530 with the foot resting on a point on the upper surface of the stainless-steel weight 415. The calipers are set to zero in this state. The reservoir 700 is filled with 0.9% saline solution and re-sealed. The outlet 740 is connected to elbow 445 via flexible plastic tubing 450.

### Sample Preparation

Use a die cutter to cut the sample at representative location (typically in the middle of the material) in a donut shape having 70 mm - R0 exterior diameter and 12 mm - Ri interior diameter.

The annular sample 475 of material to be tested has an outer diameter of 70 mm and an inner hole diameter of 12 mm. One suitable means of cutting the sample is to use a die cutter with sharp concentric blades.

The top plate 410 is lifted enough to insert the sample 475 between the top plate and the bottom plate 405 with the sample centered on the bottom plate and the plates aligned. The stop- cock 735 is opened and the level of fluid in tube 460 is set to the scribed mark 470 by adjusting the height of the reservoir 700 using the jack 705 and by adjusting the position of the tube 715 in the reservoir.

Let the saline solution flow through the sample for pre-swelling for 30 minutes, making sure that the liquid column stays at the scribed mark 470.

After the 30 min pre-swelling and when the fluid level in the tube 460 is stable at the scribed mark 470 initiate recording data from the balance and calipers by the computer. Balance readings and time elapsed are recorded every 10 seconds for five minutes. The average sample thickness B is calculated from all caliper reading between 60 seconds and 300 seconds and ex- pressed in cm. The flow rate in grams per second is the slope calculated by linear least squares regression fit of the balance reading (dependent variable) at different times (independent variable) considering only the readings between 60 seconds and 300 seconds.

Permeability k is then calculated by the following equation: $k = \frac{\left(Q/{p}_{l}\right) ⋅ μ ⋅ ln \left({R}_{0}/{R}_{i}\right)}{2 π ⋅ B ⋅ Δ p}$ (1) Where:
k is the permeability (cm²);
Q is the flow rate (g/s);
ρ₁ is the liquid density at 20°C (g/cm³); µ is the liquid viscosity at 20 °C (Pa·s); R₀ is the outer sample radius (cm);
Rᵢ is the inner sample radius (cm);
B is the average sample thickness (cm); and
Δp is the pressure drop (Pa) calculated according to the following equation: $Δ p = \left(Δh-\frac{B}{2}\right) ⋅ g ⋅ {ρ}_{l}$ (2) Where:
   Δh is the height of the fluid level in the tube 460 from the top side of the bottom plate 405 to the mark 470; Δh is 10cm +-0.1cm- (cm);
   g is the acceleration constant (m/sec²); and ρ₁ is the liquid density (g/cm³). Misc.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A personal hygiene absorbent article (20) extending in a longitudinal direction (y) and a transversal direction (x), the absorbent article comprising:
- a liquid permeable topsheet (24);
- a backsheet (25);
- an absorbent core (28) between the topsheet and the backsheet, the absorbent core comprising a core wrap having a top layer (45) and a bottom layer (46), an absorbent layer (30) between the core wrap top layer and the core wrap bottom layer, wherein the absorbent layer comprises or consists of superabsorbent polymers;
- an acquisition layer (52) disposed between the topsheet and the absorbent core;
- a cushion layer (60, 60'), wherein the cushion layer (60) is either disposed between the absorbent core and the backsheet, or the cushion layer (60') is disposed between the absorbent layer and the core wrap bottom layer;
wherein the acquisition layer and the cushion layer are made of the same bi-functional material.

2. The absorbent article according to claim 1, wherein the acquisition layer and the cushion layer have the same length as measured in the longitudinal direction.

3. The absorbent article according to claim 1 or 2, wherein the acquisition layer and the cushion layer are shorter in length than the core wrap.

4. The absorbent article according to any of the preceding claims, wherein the bi-functional material is a nonwoven having a basis weight between 14 g/m² and 100 g/m².

5. The absorbent article according to any of the preceding claims, wherein the bi-functional material has a Caliper @2.1 kPa of at least 0.3 mm, preferably from 0.4 mm to 2 mm, and more preferably from 0.5 mm and 1 mm, as measured by the Caliper Test Method disclosed herein.

6. The absorbent article according to any of the preceding claims, wherein the bi-functional material is selected from a resin bonded carded nonwoven, a spunlace nonwoven or an air-through bonded carded nonwoven, preferably wherein the bi-functional material has been hydrophillically treated.

7. The absorbent article according to any of the preceding claims, wherein the absorbent layer consists of superabsorbent polymer particles that are immobilized within the core wrap by a microfibrous glue, optionally the absorbent layer consisting of two sublayers of superabsorbent particles each sublayer immobilized by a microfibrous glue on the top layer and bottom layer of the core wrap respectively.

8. The absorbent article according to any of the preceding claims, wherein the bi-functional material has a Z-Compliance index of at least 10 mm³/N , more preferably between 15 mm³/N and 60 mm³/N, even more preferably between 25 mm³/N and 50 mm³/N, as measured by the Z-Compliance Index and Percent Recovery Measurement Method disclosed therein.

9. The absorbent article according to any of the preceding claims, wherein the bi-functional material has a Recovery from compliance index of at least 40%, preferably at least 60%, more preferably of at least 70% and up to 100%, as measured by the Z-Compliance Index and Percent Recovery Measurement Method disclosed therein.

10. The absorbent article according to any of the preceding claims, wherein the bi-functional material has IPRP of at least 5,000 SFC, preferably between 6,000 SFC and 30,000 SFC, more preferably between 8,000 SFC and 20,000 SFC (1 SFC = 10⁶ cm/Pa sec), as measured by the In-Plane Radial Permeability (IPRP) Test Method disclosed herein.

11. A continuous process for making absorbent articles according to any of the preceding claims in a converting line having a machine direction (MD) and a cross-direction (CD), said process comprising the steps of:
providing a web of bi-functional material;
slitting the web of bi-functional material along its length to form a first sub-web of acquisition material and a second sub-web of cushion material;
cutting the acquisition material and cushion material in cross-direction into discrete acquisition layers and cushion layers;
assembling the acquisition layers and cushion layers with the topsheet, backsheet, absorbent core and any other components of the absorbent articles to make discrete absorbent articles according to any of the preceding claims.

12. The continuous process according to the preceding claim, wherein the bi-functional material is continuously slit by a slitting device (70) comprising a circular blade (78) and an anvil (89) system.

13. The continuous process according to any of the preceding process claims, wherein one of the acquisition material or the cushion material passes through a flipping unit (106) to change its orientation so that the same side of the first sub-web and second sub-web faces towards the absorbent core.

14. The continuous process according to any of the preceding process claims, wherein the process comprises continuously making the absorbent cores on the same converting line.

15. The continuous process according to any of the preceding process claims, wherein the process comprises continuously making absorbent systems (114) comprising the absorbent core, the acquisition layer and cushion layer on the same line and assembling the absorbent systems with the backsheet and topsheet.
